**Europäisches Patentamt**

**European Patent Office**

**Office européen des brevets**

(19)

(11) Veröffentlichungsnummer: **0 177 520 B1**

(12) # EUROPÄISCHE PATENTSCHRIFT

(45) Veröffentlichungstag der Patentschrift:
**11.05.88**

(21) Anmeldenummer: **85901374.0**

(22) Anmeldetag: **02.04.85**

(86) Internationale Anmeldenummer:
**PCT/DE 85/00099**

(87) Internationale Veröffentlichungsnummer:
**WO 85/04569 (24.10.85** Gazette 85/23)

(51) Int. Cl.⁴: **A 61 F 13/06**

(54) **KNIE-ORTHESE.**

(30) Priorität: **05.04.84 DE 3412772**

(43) Veröffentlichungstag der Anmeldung:
**16.04.86 Patentblatt 86/16**

(45) Bekanntmachung des Hinweises auf die Patenterteilung:
**11.05.88 Patentblatt 88/19**

(84) Benannte Vertragsstaaten:
**AT BE CH DE FR GB IT LI LU NL SE**

(56) Entgegenhaltungen:
**EP - A - 0 027 172**
**DE - C - 272 865**
**FR - A - 794 292**
**US - A - 2 524 326**
**US - A - 3 375 821**
**US - A - 3 631 855**
**US - A - 4 296 744**
**US - A - 4 370 978**

(73) Patentinhaber: **KLEYLEIN, Horst, Sudetenstrasse 13, D-8502 Zirndorf (DE)**

(72) Erfinder: **KLEYLEIN, Horst, Sudetenstrasse 13, D-8502 Zirndorf (DE)**

(74) Vertreter: **Tergau, Enno et al, Hefnersplatz 3 Postfach 11 93 47, D-8500 Nürnberg 11 (DE)**

**Beschreibung**

Die Erfindung betrifft eine Knie-Orthese, welche aus einem strumpfförmigen dehnbaren Gestrick besteht, in welchem im Bereich der Kniescheibe eine ringförmige Pelotte aus elastischem inkompressiblem Werkstoff angeordnet ist.

Derartige Knie-Orthesen sind bekannt, beispielsweise aus der europäischen Patentschrift 27 172. Sie dienen zur Abstützung oder Kompression der Kniegelenk-Muskulatur sowie der dort vorhandenen Sehnenbänder sowie auch zur Lagefixierung und Abstützung der Kniescheibe. Wesentlich für die Wirksamkeit der Orthese ist die in das Gestrick eingearbeitete Pelotte, welche nach dem Vorschlag der genannten europäischen Patentschrift aus elastischem, jedoch inkompressiblem Silikon-Kautschuk oder einem Material mit gleichen Elastizitäts- und Kompressionseigenschaften bestehen soll.

Im vorliegenden Zusammenhang soll das Wort «Orthese» ein Hilfsmittel bedeuten, welches ganz allgemein die Muskeltätigkeit und Muskellage unterstützt. Das Wort «Pelotte» soll eine zusätzliche Stütze bedeuten, welche einen Druck auf Gelenkoder Muskelpartien ausübt.

Die vorbekannten Orthesen haben sich grundsätzlich bewährt. Dennoch treten in einzelnen Fällen Nachteile zutage, die beispielsweise darin bestehen, dass die Orthese beim Tragen verrutscht und dann ihre Funktion nicht mehr ausüben kann. Der Nachteil lässt sich nicht einfach dadurch beheben, dass das strumpfförmige Gestrick enger gemacht und dadurch der Anpressdruck erhöht wird, da insbesondere bei Beugung des Gelenkes eine mehr oder weniger starke Faltenbildung eintritt, wodurch es zu lokalen Druckerscheinungen und Einschnürungen kommt. Auch sind die bekannten Orthesen mit einteiliger Pelotte nicht in der Lage, die Kniegelenk-Muskulatur seitlich abzustützen sowie die Kniescheibe zu medialisieren.

Der Erfindung liegt die Aufgabe zugrunde, eine Knieorthese vorzuschlagen, deren Sitz und deren Stützwirkung über längere Zeit verbessert ist und die einen grossflächigen Druckausgleich der Kompression ohne wesentliche lokale Minderdurchblutung gewährleistet.

Die Lösung dieser Aufgabe besteht darin, dass die Pelotte seitliche Fortsätze aufweist, die sich bis in die Bereiche seitlich des Kniegelenkes erstrecken.

Aus der US-A-3 375 821 ist zwar ein Knieschutz bekannt, der aus einer strumpfförmigen dehnbaren Manschette besteht, die im Vorderteil ein Polster trägt, das beidseits mit seitlichen Polstern verbunden ist. Hier sind aber die seitlichen Polster nicht als seitliche Fortsätze des vorderen Polsters, also einteilig mit dem vorderen Polster ausgebildet.

Die Fortsätze der erfindungsgemässen Knie-Orthese werden vorteilhafterweise so weit geführt, dass sie über die Gelenkachse des Kniegelenkes greifen. Die Anordnung solcher Fortsätze erhöht einerseits die Fläche, mit der die Pelotte die Körperoberfläche berührt, so dass durch diese Massnahme die Pelotte selbst und damit auch die gesamte Orthese rutschfester sitzt. Zum anderen gestatten es die seitlichen Fortsätze, nicht nur die Kniescheibe zu fixieren, sondern auch einen Halt auf die seitlich gelegenen Muskel- und Bänderpartien auszuüben, wodurch eine wesentlich verbesserte Versorgung des Patienten erfolgt.

Als besonders vorteilhaft hat sich die Orthese bei der Therapie der Kontrapatiepatella erwiesen. Es tritt dabei eine wesentliche Verbesserung der Durchblutung des periarthikulären Gewebes ein. Die gefürchtete Atrophie wird bei der Anwendung der Orthese dadurch vermieden, dass reflektorische Inaktivitätsmuster der Muskulatur um das Kniegelenk nicht auftreten können. Soweit Ödeme sowie Ergüsse im Gelenk bestehen, werden diese schneller resorbiert, was eine Folge der ungestörten Muskelbewegung ist.

Es ist erkennbar, dass die Orthese das Kniegelenk über seinem Weichteilmantel stabilisiert, ohne dass das Gelenk in seinem natürlichen Bewegungsablauf behindert wird. Lokale Reizerscheinungen wie Druck und Minderdurchblutung werden durch den grossflächigen Druckausgleich vermieden.

Die Orthese kann auch eingesetzt werden zur Therapie von Stauungen im Kniegelenk sowie von Reizzuständen, die sich nach Verletzungen, operativen Eingriffen sowie chronisch entzündlichen Veränderungen einstellen. Besonders vorteilhaft ist die Orthese bei der Therapie der Chondropathia patellae, die gehäuft bei jungen Menschen sowie insbesondere bei Sportlern vorkommt.

Eine häufige Ursache der Chondropathia patellae ist die mediale Quadrizepsschwäche, also eine Schwäche des innenseitigen Astes des grossen vorderen Oberschenkelmuskels, die eine Dysbalance der Kniescheibenführung hervorruft. Durch die Muskelfasern des medialen Quadrizepsastes kommt diese Dysbalance relativ schnell nach Schonung des Oberschenkelmuskels bzw. des Knies vor und führt dadurch zu einer langdauernden Störung des Kniegelenkes.

Zur Verstärkung der Wirkung wird ferner vorgeschlagen, dass in die seitlichen Fortsätze zusätzliche Verstärkungen eingearbeitet sind. Diese Verstärkungen können aus elastischem aber festem Werkstoff bestehen und in die Pelotte eingegossen oder einvulkanisiert sein. Bei Versuchen des Erfinders haben sich Verstärkungsplättchen aus weichgemachtem Polyvinylchlorid bewährt, die in der angegebenen Weise in der Pelotte befestigt worden sind. Um eine sichere und innige Verbindung der Verstärkungen mit dem Pelottenwerkstoff zu erreichen, wird vorgeschlagen, dass die Verstärkungen die Form von flachen Scheiben haben, in denen sich Verankerungsöffnungen befinden, durch die der Pelottenwerkstoff fadenförmig hindurchgreift.

Schliesslich wird vorgeschlagen, dass auf der beim Gebrauch dem Körper zugewandten Seite der Pelotte im Bereich des Pelottenringes und in Nachbarschaft zu einem der Fortsätze eine wulstartige Erhöhung aus weichem, druckfestem Werkstoff angeordnet ist. Diese wulstartige Erhöhung verstärkt den Druck an der betreffenden Stelle und dient beispielsweise zur wirkungsvolleren Medialisierung der Kniescheibe. Eine derart verstärkte Orthese gestattet auch eine Verschiebung der Kniescheibe an die Aussenseite, die zum Beispiel bei Verletzungen der Kniescheibenrückfläche sinnvoll sein kann, bei denen nur eine teilweise Belastung des retropastellaren Knorpels im Rahmen der postoperativen Frühmobilisierung erlaubt ist.

Die beschriebene Knieorthese besteht in bekannter Weise aus einem strumpfförmigen, dehnbaren Gestrick aus Gummi- und/oder Kunststofffäden. Die eingearbeitete Pelotte wird vorteilhafterweise aus elastischem, jedoch inkompressiblem Silikon-Kautschuk hergestellt. Die Pelotte ist etwa 5-7 mm dick und die Ringbreite beträgt etwa 25-30 mm. Die Mittelöffnung der ringförmigen Pelotte wird vorzugsweise oval gemacht mit einer Breite von etwa 50 mm und einer Höhe von etwa 70 mm. Die seitlichen Fortsätze haben eine Höhe, die der Höhe der Ringöffnung entspricht und eine seitliche Ausladung von beispielsweise 30 mm. Dabei muss betont werden, dass die Ausladung auch grösser gemacht werden kann, je nachdem es der Einzelfall erfordert.

Eine Vergrösserung der seitlichen Ausladung der Fortsätze ist insbesondere dann angezeigt, wenn die Pelotte mit ihren Fortsätzen über die Gelenkachse des Kniegelenkes herausragen soll. In diesem Fall kann die Ausladung (Breite) an jeder Seite bis zu 70 mm betragen.

Die vorgeschlagene Pelotte wird vorzugsweise nach dem Giessverfahren aus Silikon-Kautschuk hergestellt. Der Guss erfolgt beispielsweise in einer Gipsform, welche nach Mass entsprechend den individuellen Erfordernissen des Patienten hergestellt worden ist.

Beim Herstellen der Pelotte in dieser Weise ist es denn auch leicht möglich, die zusätzlichen Verstärkungsplatten mit einzugiessen. Um eine innige Verbindung zwischen den Platten und dem Pelottenwerkstoff zu erzielen, werden die Platten an mehreren Stellen durchbohrt und dann in den flüssigen Werkstoff, der sich bereits in der Giessform befindet, eingelegt. Der Werkstoff tritt dabei durch die Öffnungen hindurch, so dass sich beim Aushärten fadenähnliche Brücken bilden, welche durch die Verstärkungsplatte hindurchgreifen.

Die Erfindung wird im folgenden anhand der beigefügten Zeichnung näher erläutert. In der Zeichnung sind Ausführungsbeispiele in schematischer Weise wiedergegeben.

Es stellen dar:

Fig. 1 eine schematisierte Seitenansicht eines Beinteiles mit angelegter Orthese;

Fig. 2 eine Draufsicht auf eine Ausführungsform der flachgelegten Pelotte;

Fig. 3 ein Querschnitt entlang der Linie III-III der Fig. 2;

Fig. 4 ein vergrösserter Ausschnitt aus Fig. 3;

Fig. 5 eine Draufsicht auf eine flachgelegte Pelotte von der Unterseite her.

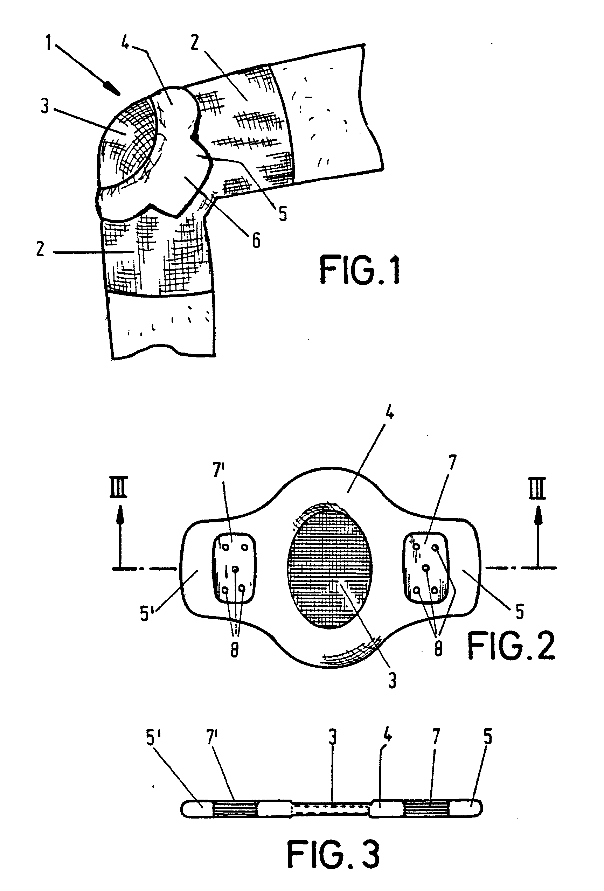

Die in der Zeichnung dargestellte Orthese ist als Ganzes mit 1 bezeichnet. Sie besteht in bekannter Weise aus einem strumpfförmigen, dehnbaren Gestrick 2, in welchem im Bereich der Kniescheibe 3 eine ringförmige Pelotte 4 aus elastischem inkompressiblem Werkstoff angeordnet ist. Die Pelotte ist mit seitlichen Fortsätzen 5, 5' ausgerüstet, die sich bis in die Bereiche seitlich des Kniegelenkes erstrecken. Wie Fig. 1 zeigt, erstrecken sich diese seitlichen Fortsätze sogar über die Gelenkachse 6 des Kniegelenkes hinaus.

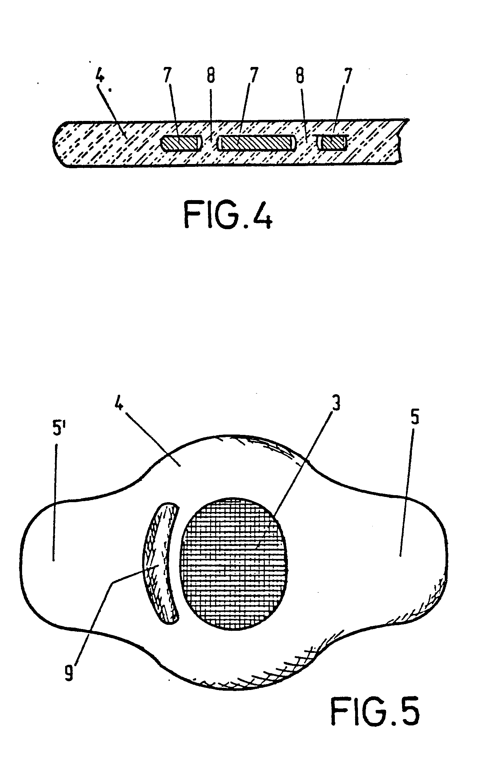

Zur Erhöhung des Seitendruckes, den die Pelotte ausübt, sind, wie die Fig. 2 und 3 zeigen, in die seitlichen Fortsätze Verstärkungen 7 und 7' eingearbeitet. Die Verstärkungen bestehen vorzugsweise aus elastischem aber festem Werkstoff, und sie sind in die Pelotte eingegossen oder einvulkanisiert. Bei anderen Ausführungsformen ist es aber auch möglich, die Verstärkungen durch Einbau von Gewebestücken und ähnlichem oder durch Einarbeiten von komprimierten Zonen zu erzeugen. Als vorteilhaft hat es sich jedoch erwiesen, wenn die Verstärkungen die Form von flachen Scheiben haben, in denen sich Verankerungsöffnungen 8 befinden, durch die der Pelottenwerkstoff fadenförmig hindurchgreift. Diese Anordnung ist aus den Fig. 2 und 3 erkennbar; sie ist jedoch in vergrösserter Form in Fig. 4 dargestellt.

In Fig. 4 ist die Pelotte 4 schraffiert wiedergegeben. Eingelegt in die Pelotte sind scheibenförmige Verstärkungen 7 (in Fig. 4 ist nur eine derartige Scheibe wiedergegeben), in der sich die erwähnten Verankerungsöffnungen 8 befinden. Da in dem in Fig. 1 gezeigten Zustand die Verankerung bereits erfolgt ist, ist der Werkstoff der Pelotte 4 durch die Öffnungen 8 hindurchgetreten und hat die bereits erwähnten fadenförmigen Halterungsgebilde erzeugt.

In Fig. 5 ist die Pelotte 4 in Unteransicht dargestellt. Dem Betrachter zugewandt ist folglich diejenige Seite der Pelotte, die bei Gebrauch am Körper des Patienten anliegt. Es ist erkennbar, dass bei dieser Ausführungsform im Bereich des Pelottenringes und in Nachbarschaft zu einem der Fortsätze 5, 5' eine wulstartige Erhöhung 9 angeordnet ist. Diese Erhöhung besteht aus weichem, druckfestem Werkstoff, beispielsweise ebenfalls aus weichgemachtem Polyvinylchlorid, oder aber auch aus Silikongummi entsprechender Provenienz. Die wulstartige Erhöhung ist beim Giessen der Pelotte gleich mit erzeugt worden; sie kann aber auch nachträglich aufgeklebt oder sonstwie befestigt werden.

## Patentansprüche

1. Knie-Orthese, bestehend aus einem strumpfförmigen dehnbaren Gestrick mit im Bereich der Kniescheibe angeordneter ringförmiger Pelotte aus elastischem inkompressiblem Werkstoff, dadurch gekennzeichnet, dass die Pelotte (4) seitliche Fortsätze (5, 5') aufweist, die sich bis in die Bereiche seitlich des Kniegelenkes erstrecken.

2. Knie-Orthese nach Anspruch 1, dadurch gekennzeichnet, dass sich die seitlichen Fortsätze (5, 5') bis über die Gelenkachse (6) des Kniegelenkes erstrecken.

3. Knie-Orthese nach Anspruch 1 oder 2, dadurch gekennzeichnet, dass in die seitlichen Fortsätze (5, 5') Verstärkungen (7, 7') eingearbeitet sind.

4. Knie-Orthese nach Anspruch 3, dadurch gekennzeichnet, dass die Verstärkungen (7, 7') aus elastischem aber festem Werkstoff bestehen und in die Pelotte eingegossen oder einvulkanisiert sind.

5. Knie-Orthese nach Anspruch 3 oder 4, dadurch gekennzeichnet, dass die Verstärkungen (7, 7') die Form von flachen Scheiben haben, in denen sich Verankerungs-Öffnungen (8) befinden, durch die der Pelotten-Werkstoff fadenförmig hindurchgreift.

6. Knie-Orthese nach einem der vorausgehenden Ansprüche, dadurch gekennzeichnet, dass auf der beim Gebrauch dem Körper zugewandten Seite der Pelotte (4) im Bereich des Pelottenringes und in Nachbarschaft zu einem der Fortsätze (5, 5') eine wulstartige Erhöhung (9) aus weichem, druckfestem Werkstoff angeordnet ist.

## Claims

1. Knee orthosis, consisting of a stocking-like stretchable knitted fabric with an annular pad, made of elastic incompressible material, disposed in the area of the patella, characterized by the improvement that the pad (4) has lateral extensions (5, 5') extending up to the areas beside the knee joint.

2. Knee orthosis according to claim 1, characterized by the improvement that the lateral extensions (5, 5') extend over the axis (6) of the knee joint.

3. Knee orthosis according to claims 1 or 2, characterized by the improvement that reinforcements (7, 7') are incorporated into the lateral extensions (5, 5').

4. Knee orthosis according to claim 3, characterized by the improvement that the reinforcements (7, 7') are made of elastic, but firm material and are cast or vulcanized into the pad.

5. Knee orthosis according to claims 3 or 4, characterized by the improvement that the reinforcements (7, 7') have the shape of flat disks provided with anchoring holes (8) through which filaments of the pad material are passed.

6. Knee orthosis according to one of the before mentioned claims, characterized by the improvement that on the side of the pad (4) which when used faces the body a bulgy raised part (9) made of soft, compression-proof material is arranged in the area of the pad ring and in the vicinity of one of the extensions (5, 5').

## Revendications

1. Orthèse du genou, constituée par un tissu tricot élastique en forme de bas, avec une pelotte annulaire en matière élastique et incompressible disposée dans la zone de la rotule, caractérisée en ce que la pelotte (4) est munie d'extensions latérales (5, 5') qui s'étendent jusqu'aux zones sur les deux côtés de l'articulation du genou.

2. Orthèse du genou selon la revendication 1, caractérisée en ce que les extensions latérales (5, 5') s'étendent jusqu'au-delà de l'axe (6) de l'articulation du genou.

3. Orthèse du genou selon l'une des revendications 1 ou 2, caractérisée en ce que des renforcements (7, 7') sont pratiqués dans les extensions latérales (5, 5').

4. Orthèse du genou selon la revendication 3, caractérisée en ce que les renforcements (7, 7') consistent en une matière élastique, mais solide, et qu'ils sont moulés ou vulcanisés dans la pelotte.

5. Orthèse du genou selon l'une des revendications 3 ou 4, caractérisée en ce que les renforcements (7, 7') ont la forme de disques plats munis d'ouvertures d'ancrage (8), à travers desquelles passe la matière de la pelotte en forme de fils.

6. Orthèse du genou selon l'une des revendications précédentes, caractérisée en ce que sur le côté de la pelotte (4) tourné en utilisation vers le corps, est disposée une élévation à bourrelet (9) faite d'une matière molle et résistante à la pression, ladite élévation se trouvant dans la zone de l'anneau de la pelotte et avoisinant l'une des extensions (5, 5').

FIG.1

FIG.2

FIG.3

FIG.4

FIG.5